# EUROPÄISCHE PATENTSCHRIFT

(11) **EP 0 550 804 B1**
(45) Veröffentlichungstag und Bekanntmachung des Hinweises auf die Patenterteilung: **08.02.1995**
(21) Anmeldenummer: 92118142.6
(22) Anmeldetag: 23.10.1992
(51) Int. Cl.: A61B 17/22

(54) **Vorrichtung zum Entfernen von Steinen aus menschlichen Körperhöhlen**
Device for removing calculus from human body cavities
Dispositif pour l'enlèvement des calculs des cavités du corps humain

(30) Priorität: 08.01.1992 DE 4200258
(43) Veröffentlichungstag der Anmeldung: 14.07.1993
(73) Patentinhaber: Winkler, Klaus, Dr., D-49076 Osnabrück (DE)
(72) Erfinder: Winkler, Klaus, Dr., D-49076 Osnabrück (DE)
(74) Vertreter: Habbel, Hans-Georg, Dipl.-Ing.

(56) Entgegenhaltungen:
- EP-A- 0 200 668
- EP-A- 0 465 051
- WO-A-88/09683
- DE-A- 3 616 205
- US-A- 4 997 435

## Beschreibung

Die Erfindung bezieht sich auf eine Vorrichtung zum Entfernen von Steinen aus menschlichen Körperhöhlen gemäß dem Oberbegriff des Hauptanspruches.

Es ist in der Praxis eine Vorrichtung zum Entfernen von Steinen aus dem Harnleiter bekannt, die mittels eines Zystoskopes in den Harnleiter eingeführt wird. Das Prinzip dieser Vorrichtung besteht darin, daß Schlingen oder sich spreizende Elemente um einen sich beispielsweise im Harnleiter festgesetzten Stein herumgeführt werden und dieser durch Zug- und Gewichtsbelastung bis zur Harnblase abgeführt wird. Mit dieser Vorrichtung und dem damit verbundenen Verfahren ist ein Erfassen von beispielsweise scharfkantigen, fest im Harnleiter fixierten Steinen oft nicht möglich. Gelingt dies dennoch, ist der Stein beim Herausgleiten ständig mit der Wandung des Harnleiters in Berührung, wodurch die Verletzungsgefahr desselben außerordentlich groß ist. Außerdem ist auch beim Herumführen der Hilfsmittel um den Stein eine zusätzliche mechanische Beanspruchung der Harnleiterwandung und damit eine akute Verletzungsgefahr gegeben.

In der DE 20 57 636 A1 wird eine Vorrichtung vorgeschlagen, die eine für den Patienten schmerzarme Entfernung von Konkrementen gestattet. Die Vorrichtung besteht aus einem doppelwandigen Schlauch, dessen in den Harnleiter einzuführendes freie Ende einen trichterförmig und gleichfalls doppelwandig ausgebildeten Kelch aufweist. Dieser ist während des Einführens in den Harnleiter von einem verschiebbaren Schutzschlauch umgeben, der vor dem Eindrücken eines gasförmigen oder flüssigen Mediums zwischen die Doppelwandungen zurückgezogen wird. Der dann freiliegende Kelch wird durch das einströmende Medium aufgeweitet und erhält so seine Trichterform.

Die Stirnseite des Schutzschlauches ist abgerundet und das Schlauchende im Außendurchmesser verjüngt, damit bei umschlossenem Kelch eine kantenlose Abrundung an seiner Vorderseite entsteht. Bis zur Freigabe des Kelches ist dieser im Schutzschlauch zusammengefaltet angeordnet.

Die Vorrichtung wird mittels eines Zystoskopes in den Harnleiter eingeführt. Ist die unmittelbare Steinnähe erreicht, erfolgt die Rückführung des Schutzschlauches und anschließend die Zufuhr des Druckmediums in den Kelch. Die sich nunmehr anschließende Kelchentfaltung weitet den Harnleiter schonend auf, so daß der Stein gelockert und von dem Kelch aufgenommen wird. Nach erfolgter Aufnahme des Steines wird der Druck des Mediums reduziert, wodurch die Kelchaußenwandung erschlafft und somit die Ausweitung des Harnleiters gemindert ist.

Während der Rückführung des Schlauches ist der aufgenommene Stein vollkommen von dem Kelch umschlossen.

Die DE 39 02 943 A1 beschreibt eine Vorrichtung zum Einbringen eines flüssigen Lösungsmittels in ein Körper-Hohlorgan, insbesondere in die Gallenblase, und zum Absaugen der aus Lösungsmittel und Organsekret bestehenden Flüssigkeit, sowie des durch den Auflösungsvorgang entstehenden Steinschlammes aus dem Hohlorgan mittels einer Kolbenpumpe, wobei das Organsekret und das Lösungsmittel im Anschluß daran mit Hilfe eines Abscheiders voneinander trennbar sind.

Während bei der Einrichtung gemäß der DE 20 57 636 A1 der Stein zwar geschützt in einem Kelch nach unten aus dem menschlichen Körper herausgezogen wird, wird bei der Anordnung gemäß der DE 39 02 943 das Innere des menschlichen Körpers der Behandlungsflüssigkeit unmittelbar ausgesetzt. Das gleiche ist der Fall bei der Einrichtung gemäß der DE 36 16 205.

Aus der gattungsbildenden US-A-49 97 435 ist eine Vorrichtung zum Entfernen von auflösbaren Steinen aus menschlichen Körperhöhlen bekanntgeworden, bei welcher der Fangsack durch Verdrillen von den Fangsack umgebenden Plastikstäbchen verschlossen werden soll. In der Praxis wird hierbei kein dichter Verschluß des Fangsackes erreicht, so daß dadurch Säure in den menschlichen Körper eintreten kann.

Der Erfindung liegt demgegenüber die Aufgabe zugrunde, das Auflösen des Steines in der Körperhöhle, und zwar innerhalb des Fangsackes, zu erreichen, ohne daß Lösungsflüssigkeit mit dem menschlichen Körper in Kontakt kommen kann.

Diese der Erfindung zugrundeliegende Aufgabe wird durch die Lehre des Hauptanspruches gelöst.

Vorteilhafte Ausgestaltungen sind in den Unteransprüchen erläutert.

Mit anderen Worten wird vorgeschlagen, daß beispielsweise bei einem Nierenstein ein aus säurebeständigem Material bestehender, relativ formbeständiger Fangsack, der vor seiner Einführung durch den Harnleiter in seinem Außendurchmesser verringert werden und sich dann aufweiten kann, durch den Harnleiter und die Blase bis in den Nierenkelch eingeführt wird und daß unter einem Röntgen- oder Ultraschallgerät der Nierenstein in diesen Fangsack hineinpraktiziert wird bzw. der Fangsack über den Nierenstein gestülpt wird. Der Fangsack kann vor seiner Führung durch den Harnleiter in seinem Außendurchmesser verringert werden und kann sich dann aufweiten, um den Stein aufzunehmen. Hierbei kann z. B. der Fangsack verdrillt werden oder nach seinem Einführen von außen aufgeweitet, d. h. aufgebläht werden. Sobald sich der Stein im Fangsack befindet, wird das offene Ende des Fangsackes von außen her geschlossen, über eine im Inneren des offenen Endes des Fangsackes angeord nete Verschlußblase, die von außen her über eine Katheterleitung mit Luft aufblasbar und damit der Fangsack verschließbar ist.

Ist der Fangsack dicht verschlossen, wird über eine säurebeständige Flüssigkeitszuleitung, die ebenfalls als Katheter ausgebildet ist, Säure in das Innere des Fangsackes geführt, wobei diese Säure mit dem menschlichen Körper nicht in Berührung kommen kann. Die Säure wird über eine zweite Flüssigkeitsableitung wieder abgesaugt, so daß eine Spülung des Inneren des Fangsackes und damit des Nierensteines mittels der Säure möglich wird. Der Nierenstein löst sich in der Säure relativ schnell auf, und zwar in der Flüssigkeit komplett, so daß ein Festsetzen von Partikelchen in der Flüssigkeitsableitung nicht zu befürchten ist.

Die Flüssigkeitszuleitungen und Flüssigkeitsableitungen können z. B. an jeweils eine Pumpe anschliessen, die einerseits die Flüssigkeit zum Fangsack hinpumpt und andererseits die Flüssigkeit aus dem Fangsack absaugt, so daß im Fangsack selbst kein überdruck entsteht. Als solche Pumpen können beispielsweise normale Infusionsspritzen eingesetzt werden. In gleicher Weise ist es aber auch möglich, einfache Infusionsflaschen einzusetzen, wobei die der Zuführung der Flüssigkeit zum Fangsack dienende Flasche höher als der menschliche Körper angeordnet wird und die der Ableitung dienende Infusionsflasche tiefer, so daß dadurch ohne eine Druckerhöhung in der Leitung, eine Spülung des Inneren des Fangsackes erfolgen kann.

Ist der Nierenstein entfernt, kann das Innere des Fangsackes mit neutraler Flüssigkeit oder Pufferlösung gespült werden, so daß es möglich ist, das offene Ende des Fangsackes wieder zu öffnen und dann den Fangsack aus der Körperhöhle zu entfernen.

Mit einer solchen Vorrichtung ist in sehr kurzer Zeit das Auflösen des Steines möglich, und es können in Abhängigkeit des Steinmaterials die unterschiedlichsten Säuren oder - falls erforderlich - auch alkalische Lösungen eingebracht werden.

Außerdem ist es möglich, den Fangsack in seinem Außendurchmesser von außen her zu verkleinern oder auszudehnen. Hierdurch wird das Einführen des Fangsackes durch den Harnleiter bis zur Niere erleichtert, d. h. in diesem Bereich kann der Fangsack hinsichtlich seines vollen Durchmessers verringert werden. Hat er dann den Nierenkelch erreicht, kann der Fangsack zu seinem vollem Außendurchmesser aufgedehnt werden, so daß die Aufnahme des Steines erleichtert ist. Auch ist es möglich den, aus einem relativ dünnen Material bestehenden Fangsack vor Einführen in den Harnleiter dadurch in seinem Außendurchmesser zu verringern, daß der Fangsack verdrillt wird, wobei er sich dann - beispielsweise im Nierenbecken - öffnet. Der Einsatz der beiden Möglichkeiten, nämlich einmal das Verdrillen des Fangsackes, zum anderen das Aufblasen von außen nach Einsetzen des Fangsackes, ist zusammen möglich.

Ein Ausführungsbeispiel der Erfindung wird an der rein schematischen Zeichnung nachfolgend erläutert.

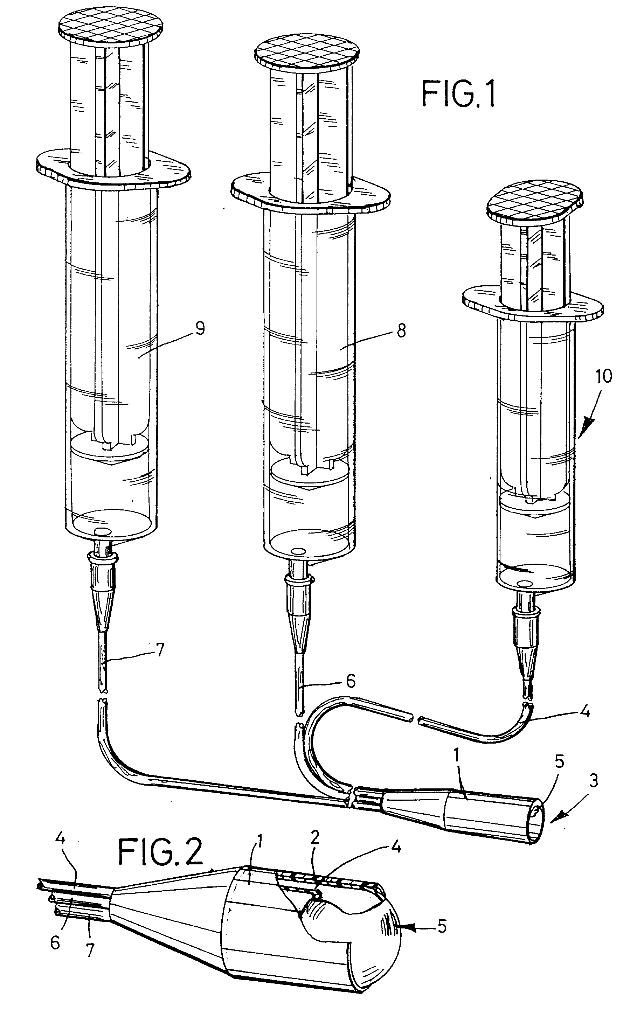

Die Zeichnung zeigt dabei in
- Fig. 1: den Aufbau der erfindungsgemäßen Vorrichtung, in
- Fig. 2: in größerem Maßstab die Ausbildung des offenen Endes des Fangsackes mit Verschlußblase, in
- Fig. 3: den Fangsack gemäß Fig. 1 in verdrilltem Zustand, in
- Fig. 3a: eine Darstellung einer "Dreifachleitung", in
- Fig. 4: einen Fangsack entsprechend der Darstellung in Fig. 2 mit einer weiteren Zuleitung einschließlich Pumpe zum Aufweiten des Durchmessers des Fangsackes nach seinem Einsetzen und in
- Fig. 5: eine abgeänderte Ausführungsform des Fangsackes.

In der Zeichnung ist mit 1 ein beispielsweise aus Kautschuk bestehender, säurebeständige Wände aufweisender Fangsack 1 dargestellt, der so klein gehalten werden kann, daß er durch den Harnleiter und die Blase bis in das Innere einer Niere, d. h. bis in den Nierenkelch einführbar ist. Während des Einführungsvorganges ist der Fangsack 1 klein gestaltet und kann in seiner Lage im Nierenkelch entfaltet oder aufgeweitet werden. Im Bereich des offenen Endes ist im Wandbereich einer Wand 2 eine Verschlußblase 5 angeordnet, die über eine Leitung 4 mit einer kleinen Pumpe 10 in Verbindung steht, über die bei Betätigen der Pumpe 10, die beispielsweise als normale Infusionsspritze ausgebildet sein kann, zu einem Aufblähen der Verschlußblase 5 führt, so daß dadurch das offene Ende 3 des Fangsackes 1 säuredicht verschlossen werden kann.

Am anderen Ende des Fangsackes 1 schließen eine Flüssigkeitszuleitung 6 und eine Flüssigkeitsableitung 7 an, die wiederum zu je einer Pumpe 8 bzw. 9 führen. Die Leitungen 4, 6 und 7 können so ausgebildet sein, daß mittels dieser Leitungen das Einführen des Fangsackes 1 in den Nierenkelch möglich ist.

Die Flüssigkeitszuleitung 6 steht mit der Pumpe 8 in Verbindung, so daß über diese Flüssigkeitszuleitung 6 eine Säure in das Innere des an seinem offenen Ende 3 durch die Verschlußblase 5 verschlossenen Fangsackes 1 eingeführt werden kann. Bei dem dargestellten Ausführungsbeispiel ist die Pumpe 8 als normale Injektionsspritze ausgebildet, die mit entsprechender Säureflüssigkeit gefüllt ist.

Die Flüssigkeitsableitung 7 steht ebenfalls mit einer z. B. als Injektionsspritze ausgebildeten Pumpe 9 in Verbindung, so daß durch diese Pumpe 9 beim Aufziehen des Kolbens die Flüssigkeit aus dem Fangsack 1 herausgesaugt werden kann. Durch wechselseitiges Betätigen der Pumpe 8 und der Pumpe 9 kann also ein ständiges Zuführen von Säureflüssigkeit und ein ständiges Abführen der Flüssigkeit aus dem Fangsack 1 herbeigeführt werden. Durch dieses Spülen des Inneren des Fangsackes 1 mit Säure kann nunmehr der im Fangsack 1 gefangene Stein aufgelöst werden. Natürlich ist es möglich, auch nur eine Leitung 7 oder 6 vorzusehen, die dann wechselseitig an eine Druck- oder Saugpumpe angeschlossen wird.

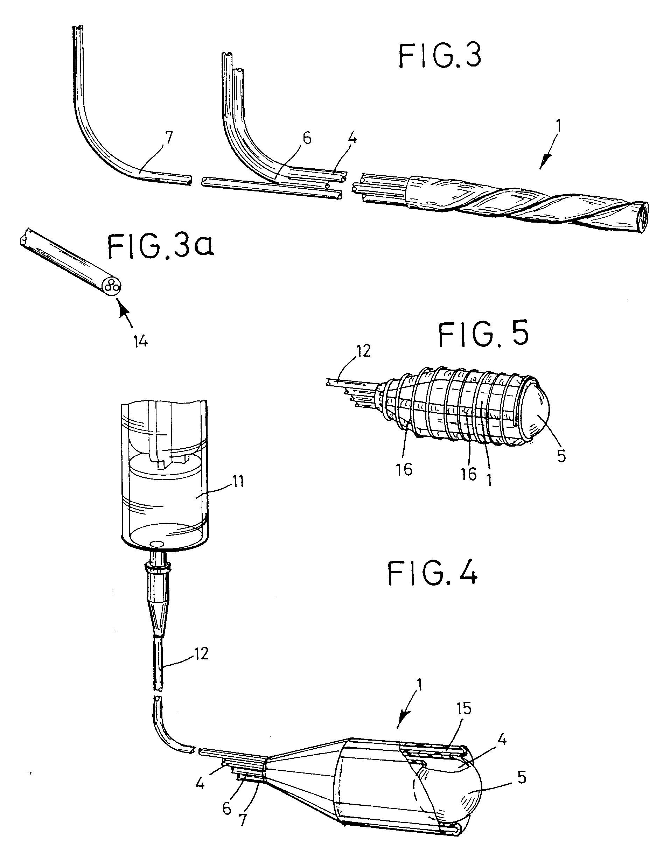

Bei der Darstellung in Fig. 3 soll verdeutlicht werden, daß durch ein Verdrillen des dünnwandigen Fangsackes 1, dessen Außendurchmesser so weit verringert werden kann, daß ein problemloses Einschieben des Fangsackes durch den Harnleiter möglich ist, wobei sich der Fangsack dann im Nierenkelch aufweitet. In der aufgeweiteten Position hat z. B. der Fangsack einen Außendurchmesser von 0,8 - 1,5 cm.

Die drei einzelnen Leitungen 4, 6 und 7 können in einer Hauptleitung 14 ausgebildet sein, wie dies Fig. 3a zeigt. Eine solche Hauptleitung hat einen Außendurchmesser von etwa 2 mm und ist daher kaum größer als eine der Leitungen 4, 6 oder 7.

Bei der Darstellung in Fig. 4 ist eine weitere Pumpe 11 vorgesehen, die über eine Leitung 12 zur Doppelwandung 15 eines Fangsackes 1 führt, so daß dadurch ein zwangsweises Kollabieren oder Aufblähen des Außendurchmessers des Fangsackes 1 vor bzw. nach Einführen 5 des Fangsackes durch den Harnleiter möglich ist. Der Fangsack kann auch gemäß Fig. 5 ausgebildet sein, d. h. mit Aufblähleitungen 16 ausgerüstet sein, die geradlinig und/oder wendelförmig an der Außenseite des Fangsackes 1 angeordnet sind.

Es ist selbstverständlich, daß die Flüssigkeitszuleitung 6 und die Flüssigkeitsableitung 7 ebenfalls säurefest ausgebildet sind.

## Patentansprüche

1. Vorrichtung zum Entfernen von auflösbaren Steinen aus menschlichen Körperhöhlen mit
a) einem säurebeständige Wände (2) aufweisenden einenendes offenen, in die Körperhöhle von außen einführbaren Fangsack (1) zur Aufnahme der Steine,
b) in den Fangsack (1) mündende, säurefeste Leitungen (6, 7) zur Zu- und Ableitung von Lösungsmitteln,
gekennzeichnet durch
c) eine im Innern des offenen Endes (3) des Fangsackes (1) vorgesehene, von außen aufblasbare Verschlußblase (5), durch die das offene Ende (3) des Fangsackes (1) flüssigkeitsdicht verschließbar ist.

2. Vorrichtung nach Anspruch 1, dadurch gekennzeichnet, daß die Leitungen (6, 7) an je eine Pumpe (8, 9) anschließen.

3. Vorrichtung nach Anspruch 1, dadurch gekennzeichnet, daß die Leitungen (6, 7) an je eine Infusionsflasche anschließen, die in unterschiedlichen Höhen angeordnet sind.

4. Vorrichtung nach Anspruch 1, dadurch gekennzeichnet, daß die Verschlußblase (5) mit einer Pumpe (10) über eine Leitung (4) verbunden ist.

5. Vorrichtung nach einem der vorhergehenden Ansprüche, dadurch gekennzeichnet, daß der eingeführte Fangsack (1) in seinem Außendurchmesser von außen her veränderbar ist.

6. Vorrichtung nach einem der vorhergehenden Ansprüche, dadurch gekennzeichnet, daß der Fangsack (1) vor Einführen in den menschlichen Körper in seinem Außendurchmesser verringerbar ist.

7. Vorrichtung nach einem der vorhergehenden Ansprüche, dadurch gekennzeichnet, daß die Leitungen (6, 7) an eine Druck- bzw. Saugpumpe anschließbar sind.

8. Vorrichtung nach einem der vorhergehenden Ansprüche, dadurch gekennzeichnet, daß die Einzelleitungen (4, 6, 7) zu einer Hauptleitung (14) zusammengefaßt sind, in der drei Einzelleitungen ausgebildet sind.

## Claims

1. A device for removing soluble stones from human body cavities, comprising
a) a collecting bag (1) open at one end, insertable into the body cavity and comprising acid-resistant walls (2), for receiving the stones,
b) acid-resistant ducts (6, 7) opening into the collecting bag (11) for supplying and carrying away solvents,
characterized by
c) a sealing pocket (5) which is provided inside the open end (3) of the collecting bag (1) and is inflatable from outside, and by means of which the open end (3) of the collecting bag (1) may be liquid-tightly sealed.

2. A device according to claim 1, characterized in that the ducts 6, 7 each connect to a pump (8, 9).

3. A device according to claim 1, characterized in that the ducts (6, 7) each connect to an infusion bottle, said infusion bottles being arranged at different heights.

4. A device according to claim 1, characterized in that the sealing pocket (5) is connected to a pump (10) via a duct (4).

5. A device according to any one of the preceding claims, characterized in that the outer diameter of the inserted collecting bag (1) may be altered from outside.

6. A device according to any one of the preceding claims, characterized in that the outer diameter of the collecting bag (1) may be reduced before insertion into the human body.

7. A device according to any one of the preceding claims, characterized in that the ducts (6, 7) may be connected to a pressure pump and a suction pump respectively.

8. A device according to any one of the preceding claims, characterized in that the individual ducts (4, 6, 7) are combined into a main duct (14), in which three individual ducts are formed.

## Revendications

1. Dispositif pour le retrait de calculs solubles de cavités du corps humain comprenant :
a) un sac de capture (1) présentant des parois (2) résistant aux acides, ouvert à une extrémité et pouvant être introduit de l'extérieur dans la cavité corporelle pour recevoir les calculs,
b) des conduites (6, 7) résistant aux acides débouchant dans le sac de capture (1) pour amener et évacuer des agents de dissolution,
caractérisé en ce qu'il comporte
c) un ballonnet de fermeture (5) prévu à l'intérieur de l'extrémité ouverte (3) du sac de capture (1) et pouvant être gonflé de l'extérieur, par lequel l'extrémité ouverte (3) du sac de capture (1) peut être fermée de manière étanche aux liquides.

2. Dispositif selon la revendication 1, caractérisé en ce que les conduites (6, 7) se raccordent chacune à une pompe (8, 9).

3. Dispositif selon la revendication 1, caractérisé en ce que les conduites (6, 7) se raccordent à des flacons de perfusion, qui sont disposés à des hauteurs différentes.

4. Dispositif selon la revendication 1, caractérisé en ce que le ballonnet de fermeture (5) est relié à une pompe (10) par une conduite (4).

5. Dispositif selon l'une ou l'ensemble des revendications précédentes, caractérisé en ce que le diamètre extérieur du sac de capture (1) peut être modifié de l'extérieur.

6. Dispositif selon l'une ou l'ensemble des revendications précédentes, caractérisé en ce que le diamètre extérieur du sac de capture (1) peut être réduit avant l'introduction dans le corps humain.

7. Dispositif selon l'une ou l'ensemble des revendications précédentes, caractérisé en ce que les conduites (6, 7) peuvent être raccordées à des pompes de pression et d'aspiration.

8. Dispositif selon l'une ou l'ensemble des revendications précédentes, caractérisé en ce que les différentes conduites (4, 6, 7) sont réunies dans une conduite principale (14) dans laquelle trois conduites distinctes sont constituées.
